# EUROPEAN PATENT APPLICATION

(11) **EP 4 487 833 A1**
(43) Date of publication of application: **08.01.2025**
(21) Application number: 23763740.0
(22) Date of filing: 03.03.2023
(51) Int. Cl.: A61K 8/42, A61K 8/35, A61K 8/34, A61K 8/44, A61Q 19/00, A61Q 19/10

(54) **PRESERVATIVE COMPRISING CAPRYLIC HYDROXAMIC ACID, 4'-HYDROXYACETOPHENONE, AND POLYOL FOR EXTERNALLY-APPLIED SKIN PREPARATION, AND COSMETIC COMPOSITION COMPRISING SAME**

(30) Priority: 04.03.2022 KR 20220028224
(71) Applicant: Activon Co., Ltd., Cheongju-si, Chungcheongbuk-do 28104 (KR)
(72) Inventor: KIM, Myo Deok, Cheongju-si, Chungcheongbuk-do 28113 (KR); CHO, Se Hee, Seoul 07691 (KR); CHO, Myung Chan, Seoul 06310 (KR); HA, Wang Su, Hwaseong-si, Gyeonggi-do 18504 (KR); PARK, Byung Gyu, Suwon-si, Gyeonggi-do 16668 (KR); CHO, Youn Ki, Yongin-si, Gyeonggi-do 17005 (KR)
(74) Representative: Tiburzi, Andrea
(86) International application number: PCT/KR2023/002945
(87) International publication number: WO 2023/167542

(57) **Abstract**

The present application relates to a preservative comprising caprylic hydroxamic acid, 4'-hydroxyacetophenone, and a polyol for an externally-applied skin preparation, and use thereof, and a preservative for an externally-applied skin preparation according to an aspect exhibits excellent anti-bacterial or anti-septic effect effects against bacteria and fungi. In particular, even though it includes 4'-hydroxyacetophenone and caprylyl glycol as active ingredients, a preservative for an externally-applied skin preparation according to an aspect does not show a deterioration in anti-bacterial or anti-septic effects against bacteria and fungi and maintains significantly excellent anti-bacterial or anti-septic effects, when applied to a cosmetic composition including a surfactant. Therefore, a preservative for an externally-applied skin preparation according to an aspect may be advantageously used for anti-bacterial, preservative, or anti-septic treatment of various cosmetic compositions, including cleaning products, or externally-applied skin preparations.

## Description

### Technical Field

The present disclosure relates to a preservative for an externally-applied skin preparation including caprylic hydroxamic acid, 4'-hydroxyacetophenone, and a polyol, and a cosmetic composition including the preservative. The present patent application claims priority to Korean Patent Application No. 10-2022-0028224, filed on March 24, 2022, the disclosure of which is incorporated by reference in its entirety.

### Background Art

Cosmetic products cannot avoid microbial contamination during a process of manufacturing or using the products, and thus preservatives capable of improving preservation of the products are essential when manufacturing cosmetic products. Examples of such preservatives that are widely used include paraben-based preservatives, imidazolidinyl urea, phenoxyethanol, and the like. However, the aforementioned preservatives have the advantages of excellent preservative activity based on high antibacterial activity, but the disadvantages of causing toxicity, skin irritation, allergy, and the like are also widely known. Thus, there is a need to develop a safe preservative with high preservative activity without causing skin irritation.

As part of this research, the development of preservatives using relatively safe ingredients such as acetophenone derivatives, caprylyl glycols, and the like continues (KR 10-2262467), but in consideration that acetophenone derivatives and caprylyl glycol may have significantly reduced antibacterial activity in an environment where surfactants are present, the inventors of the present disclosure confirmed that there are limitations in the application of acetophenone derivatives and caprylyl glycol as preservatives or antiseptic agents to an externally-applied skin preparation (e.g., a cleansing cosmetic composition) including a surfactant. Therefore, to apply acetophenone derivatives and caprylyl glycol as preservatives or antiseptic agents, there is a need to maintain excellent antibacterial activity even in an environment where surfactants are present, but there is still a lack of research on this.

### Disclosure

### Technical Problem

In order to solve the problems above, the inventors of the present disclosure developed a preservative for an externally-applied skin preparation including caprylic hydroxamic acid, 4'-hydroxyacetophenone, and a polyol, and completed the present disclosure by confirming that excellent antibacterial and antiseptic properties were observed even when the preservative for an externally-applied skin preparation was applied to a cosmetic composition including a surfactant.

An aspect of the present disclosure is to provide a preservative for an externally-applied skin preparation, including: caprylic hydroxamic acid (CHA); 4'-hydroxyacetophenone (4'-HAP); and caprylyl glycol.

Another aspect of the present disclosure is to provide a cosmetic composition including the preservative for an externally-applied skin preparation.

Another aspect of the present disclosure is to provide a method of preparing a preservative for an externally-applied skin preparation, including: mixing CHA, 4'-HAP, and caprylyl glycol.

Another aspect of the present disclosure is to provide a method of preparing a cosmetic composition, including administering the preservative for an externally-applied skin preparation.

Another aspect of the present disclosure is to provide a method of performing antibacterial, preservation, or antiseptic treatment on a cosmetic composition, including administering the preservative for an externally-applied skin preparation.

Another aspect of the present disclosure is to provide use of a composition including CHA, 4'-HAP, and caprylyl glycol, for performing antibacterial, preservation, or antiseptic treatment on an externally-applied skin preparation or a cosmetic composition.

Another aspect of the present disclosure is to provide use of a composition including CHA, 4'-HAP, and caprylyl glycol, for preparing an externally-applied skin preparation or an antibacterial or antiseptic cosmetic composition.

However, problems to be solved by the present disclosure are not limited to the above-mentioned problems, and other problems not mentioned will be clearly understood by those skilled in the art from the descriptions below.

### Technical Solution

Unless otherwise defined, all technical and scientific terms used in the present specification have the same meaning as commonly understood by those skilled in the art to which the disclosure belongs to. In general, the nomenclature used in the present specification is well known and commonly used in the art.

An aspect provides a preservative for an externally-applied skin preparation, including: caprylic hydroxamic acid (CHA); 4'-hydroxyacetophenone (4'-HAP); and caprylyl glycol.

The preservative for an externally-applied skin preparation may further include at least one polyol selected from the group consisting of glycerin, ethylene glycol, diethylene glycol, propylene glycol, dipropylene glycol, diethylene glycol, pentylene glycol, hexylene glycol, ethoxydiglycol, and C₃-C₁₂ alkanediol. The C₃-C₁₂ alkanediol may be octanediol, decanediol, propanediol, butanediol, pentanediol, hexanediol, heptanediol, undecanediol, dodecanediol, or a combination thereof, but is not limited thereto. Preferably, the preservative for an externally-applied skin preparation may further include dipropylene glycol.

According to an embodiment, the preservative for an externally-applied skin preparation including two selected from CHA, 4'-HAP, and caprylyl glycol may exhibit an antibacterial effect in a general environment, whereas its antibacterial and antiseptic effects are significantly reduced in an environment where a surfactant is present. However, the preservative for an externally-applied skin preparation including CHA, 4'-HAP, and caprylyl glycol can solve these problems and exhibit excellent antibacterial and antiseptic effects against both bacteria and fungi (yeast and mold) even in a cosmetic composition including various surfactants.

The preservative for an externally-applied skin preparation may include, based on a total weight, about 5 to 15 weight% of the CHA. In detail, the preservative for an externally-applied skin preparation may include, based on a total weight, about 5 to 10 or 10 to 15 weight% of the CHA. According to an embodiment, when the preservative for an externally-applied skin preparation includes the CHA in an amount beyond the aforementioned numerical range based on the total weight, the antibacterial or antiseptic effect may be reduced, or decreased stability, such as solidification, precipitation, decreased solubility, decreased emulsification or solubilization, or reduced safety due to skin irritation or the like may be caused.

The preservative for an externally-applied skin preparation may include, based on a total weight, about 25 to 30 weight% of the 4'-HAP. In detail, the preservative for an externally-applied skin preparation may include, based on a total weight, about 25 to 27 or 27 to 30 weight% of the 4'-HAP. According to an embodiment, when the preservative for an externally-applied skin preparation includes the 4'-HAP in an amount beyond the aforementioned numerical range based on the total weight, the antibacterial or antiseptic effect may be reduced, or decreased stability, such as solidification, precipitation, decreased solubility, decreased emulsification or solubilization, or reduced safety due to skin irritation or the like may be caused.

The preservative for an externally-applied skin preparation may include, based on a total weight, about 13 to 15 weight% of the caprylyl glycol. In detail, the preservative for an externally-applied skin preparation may include, based on a total weight, about 13 to 14 or 14 to 15 weight% of the caprylyl glycol. According to an embodiment, when the preservative for an externally-applied skin preparation includes the caprylyl glycol in an amount beyond the aforementioned numerical range based on the total weight, the antibacterial or antiseptic effect may be reduced, or decreased stability, such as solidification, precipitation, decreased solubility, decreased emulsification or solubilization, or reduced safety due to skin irritation or the like may be caused.

The preservative for an externally-applied skin preparation may include: based on a total weight, about 5 to 15, 5 to 10, or 10 to 15 weight% of the CHA; about 25 to 30, 25 to 27, or 27 to 30 weight% of the 4'-HAP; and about 13 to 15, 13 to 14, or 14 to 15 weight% of the caprylyl glycol. Preferably, the preservative for an externally-applied skin preparation may include: based on a total weight, about 5 to 15 weight%; about 25 to 30 weight% of the 4'-HAP; and about 13 to 15 weight% of the caprylyl glycol.

A weight ratio of the CHA : the 4'-HAP : the caprylyl glycol included in the preservative for an externally-applied skin preparation may be about 5 to 15, 5 to 10, or 10 to 15 : 25 to 30, 25 to 27, or 27 to 30 : 13 to 15, 13 to 14, or 14 to 15. Preferably, the weight ratio of the CHA : the 4'-HAP : the caprylyl glycol included in the preservative for an externally-applied skin preparation may be about 5 to 15 : 25 to 30 : 13 to 15.

According to an embodiment, when the weight% or weight ratio of the CHA : the 4'-HAP : the caprylyl glycol included in the preservative for an externally-applied skin preparation is within the aforementioned numerical range, the antibacterial and antiseptic effects against both bacteria and fungi (yeast and mold) may be exhibited in a cosmetic composition including various surfactants, whereas, when the weight% or weight ratio of the CHA : the 4'-HAP : the caprylyl glycol included in the preservative for an externally-applied skin preparation is beyond the aforementioned numerical range, the antibacterial and antiseptic effects against both bacteria and fungi (yeast and mold) (particularly, the antibacterial and antiseptic effects against molds) may be significantly reduced in a cosmetic composition including various surfactants.

The preservative for an externally-applied skin preparation may include at least one polyol selected from the group consisting of glycerin, ethylene glycol, diethylene glycol, propylene glycol, dipropylene glycol, diethylene glycol, pentylene glycol, hexylene glycol, ethoxydiglycol, and C₃-C₁₂ alkanediol, in an amount of about 38 to 57, 38 to 55, 38 to 50, 38 to 45, 38 to 40, 40 to 57, 40 to 55, 40 to 50, 40 to 45, 45 to 57, 45 to 55, 45 to 50, 50 to 57, 50 to 55, or 55 to 57 weight%, based on a total weight. According to an embodiment, when the preservative for an externally-applied skin preparation includes the at least one polyol in an amount beyond the aforementioned numerical range based on the total weight, the antibacterial or antiseptic effect may be reduced, or decreased stability, such as solidification, precipitation, decreased solubility, decreased emulsification or solubilization, or reduced safety due to skin irritation or the like may be caused.

The preservative for an externally-applied skin preparation may include: based on a total weight, about 5 to 15, 5 to 10, or 10 to 15 weight% of the CHA; about 25 to 30, 25 to 27, or 27 to 30 weight% of the 4'-HAP; about 13 to 15, 13 to 14, or 14 to 15 weight% of the caprylyl glycol; and about 38 to 57, 40 to 55, or 45 to 50 weight% of the at least one polyol. Preferably, the preservative for an externally-applied skin preparation may include: based on a total weight, about 5 to 15 weight%; about 25 to 30 weight% of the 4'-HAP; about 13 to 15 weight% of the caprylyl glycol; and about 38 to 57 weight% of the at least one polyol.

A weight ratio of the CHA : the 4'-HAP : the caprylyl glycol : at least one polyol included in the preservative for an externally-applied skin preparation may be about 5 to 15, 5 to 10, or 10 to 15 : 25 to 30, 25 to 27, or 27 to 30 : 13 to 15, 13 to 14, or 14 to 15 : 38 to 57, 40 to 55, or 45 to 50. Preferably, the weight ratio of the CHA : the 4'-HAP : the caprylyl glycol : at least one polyol included in the preservative for an externally-applied skin preparation may be about 5 to 15 : 25 to 30 : 13 to 15 : 38 to 57.

The preservative for an externally-applied skin preparation may further include ethylenediaminetetraacetic acid, disodium salt (EDTA 2Na).

According to an embodiment, when the preservative for an externally-applied skin preparation further includes EDTA 2Na, excellent antibacterial and antiseptic effects against both bacteria and fungi (yeast and mold) may be exhibited in a cosmetic composition including various surfactants.

The preservative for an externally-applied skin preparation may include, based on a total weight, about 0.5 to 10, 0.5 to 8, 0.5 to 5, 0.5 to 3, 0.5 to 1, 1 to 10, 1 to 8, 1 to 5, 1 to 3, 3 to 10, 3 to 8, 3 to 5, 5 to 10, 5 to 8, or 8 to 10 weight% of the EDTA 2Na.

According to an embodiment, the preservative for an externally-applied skin preparation may exhibit excellent antibacterial or antiseptic effects against various types of microorganisms (specifically, contaminant microorganisms). In detail, the preservative for an externally-applied skin preparation may inhibit growth of gram-positive bacteria, gram-negative bacteria, fungi (e.g., yeast, mold, etc.) or the like, or may kill the same. Specifically, the preservative for an externally-applied skin preparation may exhibit excellent antibacterial or antiseptic effect against *Escherichia coli, Pseudomonas aeruginosa, Staphylococcus aureus, Candida albicans, Aspergillus brasiliensis, etc.,* but is not limited thereto.

The term "externally-applied skin preparation" as used in the present specification is a concept including all compositions applied to the skin, and for example, is a concept including: various cosmetic dyes, such as basic cosmetic dyes, cosmetic dyes for makeup, cosmetic dyes for hair, etc.; and pharmaceutical compositions for topical administration, including pharmaceuticals such as such as ointments, creams, lotions, etc., and quasi-drugs.

According to an embodiment, the externally-applied skin preparation may include a surfactant. That is, the preservative for an externally-applied skin preparation may be used or applied to an externally-applied skin preparation including a surfactant, and in this case, excellent antibacterial and antiseptic effects against both bacteria and fungi (yeast and mold) may be exhibited. In other words, regarding use of the preservative for an externally-applied skin preparation, it may be used to perform antibacterial or antiseptic treatment on an externally-applied skin preparation including a surfactant. The surfactant may be a native surfactant, a synthetic surfactant, or a combination thereof. The surfactant may be a cationic surfactant, a nonionic surfactant, an anionic surfactant, a zwitterionic surfactant, or a combination thereof. The surfactant may be coco-betaine (CAS No.68424-94-2), lauryl glucoside), sodium laureth sulfate, cocamidopropyl betaine, or a combination thereof, but is not limited thereto.

According to an embodiment, the externally-applied skin preparation may include, based on a total weight, about 0.5 to 70 weight% of the surfactant, but is not limited thereto. Preferably, the amount of the surfactant included may be about 1 to 50, 10 to 40, or 20 to 40 weight%.

The externally-applied skin preparation may be a cleansing cosmetic composition.

The cleansing cosmetic composition may include shampoo, rinse, scalp cleanser, foam cleanser, soap, cleansing oil, cleansing cream, face cleanser, body cleanser, toothpaste, mouth freshener, etc., but is not limited thereto.

The term "preservative for an externally-applied skin preparation" as used in the present specification may be used interchangeably with "antibacterial agent", "preservative", "antibacterial preservative", "antiseptic preservative", "antibacterial composition", "antiseptic composition", "antibacterial or antiseptic composition", "antibacterial cosmetic composition", "antiseptic cosmetic composition", "antibacterial or antiseptic cosmetic composition", etc.

In the present specification, the term "antibacterial" as used refers to resistance to all contaminant microorganisms such as bacteria, mold, yeast, etc., the "antibacterial activity" or "antibacterial effect" refers to defensive activity or protective effect against these contaminant microorganisms, and the "antiseptic activity" or "antiseptic effect" refers to defensive activity or defense effect against decay caused by contaminant microorganisms, and is a concept that includes antibacterial activity or antibacterial effect.

In the present specification, term "preservative" as used in the present specification refers to a substance that has antiseptic or antioxidant activity and can prevent deterioration of a product for a long period of time and maintain its original state, and the "preservative activity" refers to a defensive activity that can prevent a product from deterioration for a long period of time and maintain its original state.

The preservative for an externally-applied skin preparation may additionally include an additive, such as purified water, a carrier, an emulsifier, a moisturizer, a skin conditioning agent, a surfactant, a chelating agent, an antioxidant, an antiseptic agent, a sterilizing agent, a stabilizer, a preservative, a pH adjustor, a lubricant, a solubilizer, a solvent, etc. In addition, the preservative for an externally-applied skin preparation may additionally include a substance that can supplementally provide essential nutrients to the skin or a substance that ameliorates the skin condition, such as a functional substance that has effects on wrinkles or skin-whitening. In addition, the preservative for an externally-applied skin preparation may additionally include an auxiliary agent including, but not limited thereto, natural fragrance, cosmetic fragrance, or plant extracts.

Another aspect provides a cosmetic composition including the preservative for an externally-applied skin preparation.

According to an embodiment, the cosmetic composition may include a surfactant. That is, the preservative for an externally-applied skin preparation may be used or applied to a cosmetic composition including a surfactant, and in this case, excellent antibacterial and antiseptic effects against both bacteria and fungi (yeast and mold) may be exhibited. In other words, regarding use of the preservative for an externally-applied skin preparation, it may be used to perform antibacterial or antiseptic treatment on a cosmetic composition including a surfactant. The surfactant may be a native surfactant, a synthetic surfactant, or a combination thereof. The surfactant may be a cationic surfactant, a nonionic surfactant, an anionic surfactant, a zwitterionic surfactant, or a combination thereof. The surfactant may be coco-betaine (CAS No.68424-94-2), lauryl glucoside), sodium laureth sulfate, cocamidopropyl betaine, or a combination thereof, but is not limited thereto.

According to an embodiment, the cosmetic composition may include, based on a total weight, about 0.5 to 70 weight% of the surfactant, but is not limited thereto. Preferably, the amount of the surfactant included may be about 1 to 50, 10 to 40, or 20 to 40 weight%.

The cosmetic composition may be a cleansing cosmetic composition.

The cleansing cosmetic composition may include shampoo, rinse, scalp cleanser, foam cleanser, soap, cleansing oil, cleansing cream, face cleanser, body cleanser, toothpaste, mouth freshener, etc., but is not limited thereto.

The cosmetic composition may include, based on a total weight thereof, about 0.0001 to 50 weight% of the preservative for an externally-applied skin preparation. In detail, the cosmetic composition may include the preservative for an externally-applied skin preparation in an amount of about 0.0001 to 0.001, 0.001 to 0.01, 0.01 to 0.1, 0.1 to 1, 1 to 10, 10 to 20, 20 to 30, 30 to 40, or 40 to 50 weight%, based on a total weight of the cosmetic composition. According to an embodiment, when the cosmetic composition includes the preservative for an externally-applied skin preparation in an amount less than about 0.0001 weight% based on a total weight of the cosmetic composition, the antibacterial or antiseptic effect may be reduced, whereas, when the cosmetic composition includes the preservative for an externally-applied skin preparation in an amount greater than about 50 weight% based on a total weight of the cosmetic composition, stability degradation such as solidification, precipitation, decreased solubility, decreased emulsification or decreased solubilization, etc., may be caused.

The cosmetic composition may additionally include an additive selected from the group consisting of purified water, a carrier, an emulsifier, a moisturizer, a skin conditioning agent, a surfactant, a chelating agent, an antioxidant, an antiseptic agent, a sterilizing agent, a stabilizer, a preservative, a pH adjustor, a lubricant, a solubilizer, a solvent, etc., but is not limited thereto. In addition, the cosmetic composition may additionally include a substance that can supplementally provide essential nutrients to the skin or a substance that ameliorates the skin condition, such as a functional substance that has effects on wrinkles or skin-whitening. In addition, the cosmetic composition may additionally include an auxiliary agent including, but not limited thereto, natural fragrance, cosmetic fragrance, or plant extracts.

The cosmetic composition may be prepared in any formulation commonly prepared in the art. In detail, the cosmetic composition may be formulated into a solution, an emulsion, a suspension, a softening tonic, a nourishing tonic, a massage cream, a nourishing cream, a facial pack, a gel, a cleanser, a foam cleanser, a cleansing oil, a cleansing cream, a skin adhesive type cosmetic, a lipstick, a powder, a makeup base, a foundation, a shampoo, a rinse, a scalp cleanser, a scalp toner, a scalp cream, a scalp pack, a scalp gel, a scalp ointment, a face cleanser, a body cleanser, a soap, a toothpaste, a mouthwash, a paste, a lotion, an ointment, a gel, a cream, a patch, a spray, or an aerosol, but is not limited thereto.

A carrier included in the cosmetic composition may be selectively used depending on the formulation type of a cosmetic. For example, when preparing a cosmetic in the formulation of ointment, paste, cream, or gel, a carrier ingredient, such as wax, paraffin, starch, tragacanthin, a cellulose derivative, polyethylene glycol, silicone, bentonite, silica, talc, zinc oxide, etc., may be used alone or in combination. When preparing a cosmetic in the formulation of powder or spray, a carrier ingredient may be lactose, talc, silica, aluminum hydroxide, calcium silicate, polyamide powder, chlorofluorohydrocarbon, propane/butane, dimethyl ether, etc., and may be used alone or in combination. When preparing a cosmetic in the formulation of solution or emulsion, a carrier ingredient may be water, ethanol, isopropanol, ethyl carbonate, ethyl acetate, benzyl alcohol, benzyl benzoate, propylene glycol, 1,3-butylglycol oil, cottonseed oil, peanut oil, maize germ oil, olive oil, castor oil, sesame seed oil, glycerol aliphatic ester, polyethylene glycol, fatty acid ester of sorbitan, etc., and may be used alone or in combination. When preparing a cosmetic in the formulation of suspension, a carrier ingredient may be water, ethanol or propylene glycol, ethoxylated isostearyl alcohol, polyoxyethylene sorbitol ester, polyoxyethylene sorbitan ester, microcrystalline cellulose, aluminum metahydroxide, bentonite, agar, tragacanthin, etc., and may be used alone or in combination. When preparing a cosmetic in the formulation of soap, a carrier ingredient may be an alkali metal salt of fatty acid, a salt of fatty acid hemiester, a fatty acid protein hydrolysate, isethionate, a lanolin derivative, aliphatic alcohol, vegetable oil, glycerol, a sugar, etc., and may be used alone or in combination.

Among the terms or elements mentioned regarding the cosmetic composition, those mentioned in the description of the preservative for an externally-applied skin preparation are understood to be the same as previously mentioned in the description of the preservative for an externally-applied skin preparation.

Another aspect provides a method of preparing a preservative for an externally-applied skin preparation, including: mixing CHA, 4'-HAP, and caprylyl glycol.

The method may further include adding and mixing at least one polyol selected from the group consisting of glycerin, ethylene glycol, diethylene glycol, propylene glycol, dipropylene glycol, diethylene glycol, pentylene glycol, hexylene glycol, ethoxydiglycol, and C₃-C₁₂ alkanediol.

The method may further include adding and mixing EDTA 2Na.

Another aspect provides a method of preparing a cosmetic composition, including administering the preservative for an externally-applied skin preparation.

Another aspect provides a method of performing antibacterial, preservation, or antiseptic treatment on a cosmetic composition, including administering the preservative for an externally-applied skin preparation.

In the method, a mixing ratio or weight% of each ingredient is as mentioned in the description of the preservative for an externally-applied skin preparation.

In the method, specific conditions and methods may be based on common techniques in the art, but are not limited thereto.

Among the terms or elements mentioned regarding the method , those mentioned in the description of the preservative for an externally-applied skin preparation or cosmetic composition are understood to be the same as previously mentioned in the description of the preservative for an externally-applied skin preparation or cosmetic composition.

Another aspect provides use of a composition including CHA, 4'-HAP, and caprylyl glycol, for performing antibacterial, preservation, or antiseptic treatment on an externally-applied skin preparation or a cosmetic composition.

Another aspect provides use of a composition including CHA, 4'-HAP, and caprylyl glycol, for preparing an externally-applied skin preparation or an antibacterial or antiseptic cosmetic composition.

The composition may further include at least one polyol selected from the group consisting of glycerin, ethylene glycol, diethylene glycol, propylene glycol, dipropylene glycol, diethylene glycol, pentylene glycol, hexylene glycol, ethoxydiglycol, and C₃-C₁₂ alkanediol.

The composition may further include EDTA 2Na.

A mixing ratio or weight% of each ingredient is as mentioned in the description of the preservative for an externally-applied skin preparation.

Among the terms or elements mentioned regarding the use, those mentioned in the description of the preservative for an externally-applied skin preparation, cosmetic composition, or methods are understood to be the same as previously mentioned in the description of the preservative for an externally-applied skin preparation, cosmetic composition, or methods.

### Advantageous Effects

A preservative for an externally-applied skin preparation according to an aspect exhibits an excellent antibacterial or antiseptic effect against bacteria and fungi, and in particular, even when applied to a cosmetic composition including a surfactant, the antibacterial or antiseptic effect against bacteria and fungi is not reduced, and is still significantly excellent.

### Mode for Invention

Hereinafter, the present disclosure will be described in detail with reference to Examples and Experimental Examples below. However, these Examples and Experimental Examples are provided for illustrative purposes only, and the scope of the present disclosure is not limited thereto.

### 1. Preparation and evaluation of preservative for externally-applied skin preparation

### Example 1. - Example 10. Preparation of preservative for externally-applied skin preparation

In this example, a preservative for an externally-applied skin preparation including: caprylic hydroxamic acid (CHA); 4'-hydroxyacetophenone (4'-HAP); and caprylyl glycol was prepared.

In detail, a preservative for an externally-applied skin preparation in which CHA, 4'-HAP, caprylyl glycol, and dipropylene glycol were mixed and stirred while raising the temperature to about 60 to 70 °C was prepared. Here, 8 different preservatives for an externally-applied skin preparation (Examples 1 to 8) were obtained, varying in the weight% of each ingredient in the mixture. In addition, a preservative for an externally-applied skin preparation in which EDTA 2Na was additionally added and mixed, and stirred while raising the temperature to about 60 to 70 °C was prepared. Here, 2 different preservatives for an externally-applied skin preparation (Examples 9 and 10) were obtained, varying in the weight% of each ingredient in the mixture. Furthermore, 3 different preservatives for an externally-applied skin preparation of Comparative Examples (Comparative Examples 1 to 3) including two ingredients selected from CHA, 4'-HAP, and caprylyl glycol and dipropylene glycol were obtained, and 5 different preservatives for an externally-applied skin preparation of Comparative Examples (Comparative Examples 4 to 8) including CHA, 4'-HAP, caprylyl glycol, and dipropylene glycol were obtained, wherein the content of each ingredient was different from the content of each ingredient in the preservative for an externally-applied skin preparation according to Examples above. The preservatives of Comparative Examples were prepared by the same method as those of Examples. The ingredients of the prepared preservatives of Examples 1 to 10 and Comparative Examples 1 to 8 are as shown in Tables 1 and 2.

**[Table 1]**

| Ingredient (unit: weight%) | Example 1 | Example 2 | Example 3 | Example 4 | Example 5 | Example 6 | Example 7 | Example 8 | Example 9 | Example 10 |
|---|---|---|---|---|---|---|---|---|---|---|
| CHA | 5 | 5 | 5 | 5 | 10 | 10 | 15 | 15 | 15 | 15 |
| 4-HAP | 25 | 30 | 25 | 30 | 25 | 30 | 25 | 30 | 25 | 30 |
| Caprylyl glycol | 13 | 13 | 15 | 15 | 13 | 15 | 13 | 15 | 13 | 15 |
| Dipropylene glycol | 57 | 52 | 55 | 50 | 52 | 45 | 47 | 40 | 45 | 38 |
| EDTA 2Na | - | - | - | - | - | - | - | - | 2 | 2 |

**[Table 2]**

| Ingredient (unit: weight%) | Comparative Example 1 | Comparative Example 2 | Comparative Example 3 | Comparative Example 4 | Comparative Example 5 | Comparative Example 6 | Comparative Example 7 | Comparative Example 8 |
|---|---|---|---|---|---|---|---|---|
| CHA | - | 15 | 15 | 3 | 20 | 20 | 5 | 20 |
| 4-HAP | 30 | 30 | | 30 | 30 | 35 | 20 | 35 |
| Caprylyl glycol | 15 | | 15 | 15 | 15 | 15 | 10 | 20 |
| Dipropylene glycol | 55 | 55 | 70 | 52 | 35 | 30 | 65 | 25 |
| EDTA 2Na | - | - | - | - | - | - | - | - |

### Experimental Example 1. Confirmation of antibacterial effect of preservative for an externally-applied skin preparation in the presence of cationic surfactant

The antibacterial effect of the prepared preservatives for an externally-applied skin preparation of Examples 1 to 10 was confirmed in the presence of a cationic surfactant. For use as a cationic surfactant, Coco-Betaine (CAS No.68424-94-2) which is the most commonly used cationic surfactant in the cosmetics field was used.

In detail, as shown in Tables 3 and 4, each of the prepared preservatives for an externally-applied skin preparation of Examples 1 to 10 was mixed with a cationic surfactant, and then, it was confirmed whether the resulting mixture has an antibacterial effect against bacteria, yeast, and molds. To confirm an antibacterial effect against bacteria, cultures of *Escherichia coli* (ATCC 8739), *Pseudomonas aeruginosa* (ATCC 9027), and *Staphylococcus aureus* (ATCC 6538) were mixed at a ratio of 1:1:1, and the mixed culture was inoculated into each of the mixtures listed in Tables 3 and 4. Here, the inoculation was done so that the number of inoculated bacteria was about 10⁶ CFU/g. To confirm an antibacterial effect against yeast, a culture of *Candida albicans* (ATCC 10231) was inoculated into each of the mixtures listed in Tables 3 and 4. Here, the inoculation was done so that the number of inoculated bacteria was about 10⁵ CFU/g. To confirm an antibacterial effect against molds, a culture of *Aspergillus brasiliensis* (ATCC 16404) was inoculated into each of the mixtures listed in Tables 3 and 4. Here, the inoculation was done so that the number of inoculated bacteria was about 10⁵ CFU/g. For 7 days after the inoculation, the samples were stored at room temperature (about 25 °C), and the number of viable bacteria in each sample was measured to finally analyze the death rate (%) of bacteria. The results are shown in Table 5. The death rate (%) of bacterial was calculated by the following formula: Death rate (%): (number of inoculated bacteria - number of viable bacteria after storage at about 25 °C for 7 days) / (number of inoculated bacteria) x 100.

Regarding an aqueous solution containing only a cationic surfactant at a concentration of about 30 % without adding any preservative for an externally-applied skin preparation prepared according to Examples 1 to 10 and Comparative Examples 1 to 8, the death rate (%) was measured, and this aqueous solution was used as Control group 1. Also, regarding an aqueous solution containing only the preservative for an externally-applied skin preparation prepared according to Comparative Example 1 at a concentration of 1 % without adding any cationic surfactant, the death rate (%) was measured, and this aqueous solution was used as Control group 2. Furthermore, each of the preservatives for an externally-applied skin preparation prepared according to Comparative Examples 1 to 8 was mixed with a cationic surfactant, and the death rate (%) in the resulting mixture was measured. These mixtures were used as Control groups 3 to 10, respectively.

**[Table 3]**

| Ingredient (unit: weight%) | | Mixture | | | | | | | | | | | | |
|---|---|---|---|---|---|---|---|---|---|---|---|---|---|---|
| | | Control group | | | Experimental group | | | | | | | | | |
| | | 1 | 2 | 3 | 1 | 2 | 3 | 4 | 5 | 6 | 7 | 8 | 9 | 10 |
| Distilled water | | Upto 100 | | | | | | | | | | | | |
| Cationic surfactant | Coco-Betaine | 30 | - | 30 | 30 | 30 | 30 | 30 | 30 | 30 | 30 | 30 | 30 | 30 |

| Comparative Example | 1 | - | 1 | 1 | - | - | - | - | - | - | - | - | - | - |
|---|---|---|---|---|---|---|---|---|---|---|---|---|---|---|
| | 1 | - | - | - | 1 | - | - | - | - | - | - | - | - | - |
| | 2 | - | - | - | - | 1 | - | - | - | | - | - | - | - |
| | 3 | - | - | - | - | - | 1 | - | - | - | - | - | - | - |
| | 4 | - | - | - | - | - | - | 1 | - | - | - | - | - | - |
| Example | 5 | - | - | - | - | - | - | - | 1 | - | - | - | - | - |
| | 6 | - | - | - | - | - | - | - | - | 1 | - | - | - | - |
| | 7 | - | - | - | - | - | - | - | - | - | 1 | - | - | - |
| | 8 | - | - | - | - | - | - | - | - | - | - | 1 | - | - |
| | 9 | - | - | - | - | - | - | - | - | - | - | - | 1 | - |
| | 10 | - | - | - | - | - | - | - | - | - | - | - | - | 1 |

**[Table 4]**

| Ingredient (unit: weight%) | | Mixture | | | | | | |
|---|---|---|---|---|---|---|---|---|
| | | Control group | | | | | | |
| | | 4 | 5 | 6 | 7 | 8 | 9 | 10 |
| Distilled water | | Upto 100 | | | | | | |
| Cationic surfactant | Coco-Betaine | 30 | 30 | 30 | 30 | 30 | 30 | 30 |
| Comparative Example | 2 | 1 | - | - | - | - | | |
| | 3 | - | 1 | - | - | - | | |
| | 4 | - | | 1 | - | - | | |
| | 5 | - | | - | 1 | - | | |
| | 6 | - | | - | - | 1 | | |
| | 7 | - | | - | - | - | 1 | |
| | 8 | - | | - | - | - | - | 1 |

Consequently, as shown in Table 5, it was confirmed that the antibacterial effect against both bacteria and fungi (yeast and molds) was at a significantly low level when only the cationic surfactant was present (Control group 1). Also, Comparative Example 1 including 4'-HAP and caprylyl glycol was found to exhibit the antibacterial effect moderately in an environment where a surfactant was absent (Control group 2), whereas it exhibited significantly reduced antibacterial effect in an environment where a cationic surfactant was present (Control group 3). Meanwhile, Examples 1 to 10 further including CHA in addition to Comparative Example 1 were found to exhibit the significantly excellent antibacterial effect with a death rate of about 99 % or more against both bacteria and fungi (yeast and molds), even in an environment where a cationic surfactant was present (Experimental groups 1 to 10). In particular, among Examples, Examples 9 and 10 further including EDTA 2Na were found to exhibit the most excellent antibacterial effect with a death rate of about 99.99 % against both bacteria and fungi (yeast and molds), even in an environment where a cationic surfactant was present (Experimental groups 9 and 10). Also, Comparative Examples 2 and 3 including CHA and any one of 4'-HAP and caprylyl glycol were found to exhibit the significantly reduced antibacterial effect (especially against fungi (yeast and molds)), in an environment where a cationic surfactant was present (Control groups 4 and 5).

Furthermore, as shown in Table 5, only when CHA, 4'-HAP, and caprylyl glycol were included at a certain content, it was confirmed that the excellent antibacterial effect was exhibited against both bacteria and fungi (yeast and molds), even in an environment where a cationic surfactant was present. In detail, only the preservative for an externally-applied skin preparation of Example including about 5 to 15 wt% of CHA, about 25 to 30 wt% of 4'-HAP, and about 13 to 15 wt% of caprylyl glycol was found to exhibit the excellent antibacterial effect against both bacteria and fungi (yeast and molds), even in an environment where a cationic surfactant was present. Meanwhile, Comparative Example in which the contents of CHA, 4'-HAP, and caprylyl glycol were beyond the aforementioned numerical ranges was found to exhibit the significantly reduced antibacterial effect (especially, against fungi (yeast and molds)), in an environment where a cationic surfactant was present.

**[Table 5]**

| Death rate (%) | | | |
|---|---|---|---|
| | Bacteria | Yeast | Mold |
| Control group 1 | 55.333 | 50.215 | 10.032 |
| Control group 2 | 99.9999 | 99.999 | 99.2 |
| Control group 3 | 59.3154 | 69.875 | 33.992 |
| Experimental group 1 | 99.9879 | 99.999 | 99.333 |
| Experimental group 2 | 99.9929 | 99.999 | 99.267 |
| Experimental group 3 | 99.9936 | 99.999 | 99.4 |
| Experimental group 4 | 99.995 | 99.999 | 99.2 |
| Experimental group 5 | 99.9943 | 99.999 | 99.733 |
| Experimental group 6 | 99.9943 | 99.999 | 99.947 |
| Experimental group 7 | 99.9999 | 99.999 | 99.997 |
| Experimental group 8 | 99.9999 | 99.999 | 99.998 |
| Experimental group 9 | 99.9999 | 99.999 | 99.999 |
| Experimental group 10 | 99.9999 | 99.999 | 99.999 |
| Control group 4 | 95.2857 | 97.143 | 57.333 |
| Control group 5 | 99.9107 | 99.914 | 76.667 |
| Control group 6 | 99.9071 | 96.357 | 66.667 |
| Control group 7 | 99.9714 | 99.286 | 73.333 |
| Control group 8 | 99.8857 | 97.857 | 70 |
| Control group 9 | 99.9143 | 99.929 | 95.067 |
| Control group 10 | 99.8714 | 99.893 | 95.667 |

### Experimental Example 2. Confirmation of antibacterial effect of preservative for an externally-applied skin preparation in the presence of nonionic surfactant

The antibacterial effect of the prepared preservatives for an externally-applied skin preparation of Examples 1 to 10 was confirmed in the presence of a nonionic surfactant. For use as a nonionic surfactant, lauryl glucoside which is the most commonly used nonionic surfactant in the cosmetic fields was used.

In detail, as shown in Tables 6 and 7, each of the prepared preservatives for an externally-applied skin preparation of Examples 1 to 10 was mixed with a nonionic surfactant, and then, it was confirmed whether the resulting mixture has an antibacterial effect against bacteria, yeast, and molds. Specific experimental protocols are the same as those performed in Experimental Example 1.

An aqueous solution containing only a nonionic surfactant at a concentration of about 30 % without adding any preservative for an externally-applied skin preparation prepared according to Examples 1 to 10 and Comparative Examples 1 to 8 was used as Control group 11. Also, an aqueous solution containing only the preservative for an externally-applied skin preparation prepared according to Comparative Example 1 at a concentration of 1 % without adding any nonionic surfactant was used as Control group 12. Furthermore, each of the preservatives for an externally-applied skin preparation prepared according to Comparative Examples 1 to 8 was mixed with a nonionic surfactant, and these mixtures were used as Control groups 13 to 20, respectively.

**[Table 6]**

| **Ingredient (unit: weight%)** | | **Mixture** | | | | | | | | | | | | |
|---|---|---|---|---|---|---|---|---|---|---|---|---|---|---|
| | | Control group | | | Experimental group | | | | | | | | | |
| | | 11 | 12 | 13 | 11 | 12 | 13 | 14 | 15 | 16 | 17 | 18 | 19 | 20 |
| Distiller water | | Uoto 100 | | | | | | | | | | | | |
| Nonionic surfactant | Lauryl glucoside | 30 | - | 30 | 30 | 30 | 30 | 30 | 30 | 30 | 30 | 30 | 30 | 30 |
| Comparative Example | 1 | - | 1 | 1 | - | - | - | - | - | - | - | - | - | - |
| | 1 | - | - | - | 1 | - | - | - | - | - | - | - | - | - |
| | 2 | - | - | - | - | 1 | | - | - | - | - | - | - | - |
| | 3 | - | - | - | - | - | 1 | - | - | - | - | - | - | - |
| | 4 | - | - | - | - | - | - | 1 | - | - | - | - | - | - |
| Example | 5 | - | - | - | - | - | - | - | 1 | - | - | - | - | - |
| | 6 | - | - | - | - | - | - | - | - | 1 | - | - | - | - |
| | 7 | - | - | - | - | - | - | - | - | - | 1 | - | - | - |
| | 8 | - | - | - | - | - | - | - | - | - | - | 1 | - | - |
| | 9 | - | - | - | - | - | - | - | - | - | - | - | 1 | - |
| | 10 | - | - | - | - | - | - | - | - | - | - | - | - | 1 |

**[Table 7]**

| Ingredient (unit: weight%) | | Mixture | | | | | | |
|---|---|---|---|---|---|---|---|---|
| | | Control group | | | | | | |
| | | 14 | 15 | 16 | 17 | 18 | 19 | 20 |
| Distilled water | | Upto 100 | | | | | | |
| Nonionic surfactant | Lauryl glucoside | 30 | 30 | 30 | 30 | 30 | 30 | 30 |
| Comparative Example | 2 | 1 | - | - | - | - | - | - |
| | 3 | - | 1 | - | - | - | - | - |
| | 4 | - | - | 1 | - | - | - | - |
| | 5 | - | - | - | 1 | - | - | - |
| | 6 | - | - | - | - | 1 | - | - |
| | 7 | - | - | - | - | - | 1 | - |
| | 8 | - | - | - | - | - | - | 1 |

Consequently, as shown in Table 8, it was confirmed that the antibacterial effect against both bacteria and fungi (yeast and molds) was at a significantly low level when only the nonionic surfactant was present (Control group 11). Also, Comparative Example 1 including 4'-HAP and caprylyl glycol was found to exhibit the antibacterial effect moderately in an environment where a nonionic surfactant was absent (Control group 12), whereas it exhibited the significantly reduced antibacterial effect in an environment where a nonionic surfactant was present (Control group 13). Meanwhile, Examples 1 to 10 further including CHA in addition to Comparative Example 1 were found to exhibit the significantly excellent antibacterial effect with a death rate of about 99 % or more against both bacteria and fungi (yeast and molds), even in an environment where a nonionic surfactant was present (Experimental groups 11 to 20). In particular, among Examples, Examples 9 and 10 further including EDTA 2Na were found to exhibit the most excellent antibacterial effect with a death rate of about 99.99 % against both bacteria and fungi (yeast and molds), even in an environment where a nonionic surfactant was present (Experimental groups 19 and 20). Also, Comparative Examples 2 and 3 including CHA and any one of 4'-HAP and caprylyl glycol were found to exhibit the significantly reduced antibacterial effect (especially against fungi (yeast and molds)), in an environment where a nonionic surfactant was present (Control groups 14 and 15).

Furthermore, as shown in Table 8, only when CHA, 4'-HAP, and caprylyl glycol were included at a certain content, it was confirmed that the excellent antibacterial effect was exhibited against both bacteria and fungi (yeast and molds), even in an environment where a nonionic surfactant was present. In detail, only the preservative for an externally-applied skin preparation of Example including about 5 to 15 wt% of CHA, about 25 to 30 wt% of 4'-HAP, and about 13 to 15 wt% of caprylyl glycol was found to exhibit the excellent antibacterial effect against both bacteria and fungi (yeast and molds), even in an environment where a nonionic surfactant was present. Meanwhile, Comparative Example in which the contents of CHA, 4'-HAP, and caprylyl glycol were beyond the aforementioned numerical ranges was found to exhibit the significantly reduced antibacterial effect (especially, against fungi (yeast and molds)), in an environment where a nonionic surfactant was present.

**[Table 8]**

| Death rate (%) | | | |
|---|---|---|---|
| | Bacteria | Yeast | Mold |
| Control group 11 | 50.3894 | 59.997 | 15.977 |
| Control group 12 | 99.9850 | 99.964 | 98.92 |
| Control group 13 | 60.9970 | 99.929 | 52.953 |
| Experimental group 11 | 99.9879 | 99.999 | 99.400 |
| Experimental group 12 | 99.9914 | 99.999 | 99.533 |
| Experimental group 13 | 99.9921 | 99.999 | 99.667 |
| Experimental group 14 | 99.9957 | 99.999 | 99.333 |
| Experimental group 15 | 99.9943 | 99.999 | 99.793 |
| Experimental group 16 | 99.9950 | 99.999 | 99.953 |
| Experimental group 17 | 99.9996 | 99.999 | 99.998 |
| Experimental group 18 | 99.9996 | 99.999 | 99.998 |
| Experimental group 19 | 99.9999 | 99.999 | 99.999 |
| Experimental group 20 | 99.9999 | 99.999 | 99.999 |
| Control group 14 | 96.3571 | 98.929 | 69.333 |
| Control group 15 | 99.7571 | 99.929 | 72.667 |
| Control group 16 | 99.9621 | 99.964 | 94.133 |
| Control group 17 | 99.9707 | 99.971 | 88.000 |
| Control group 18 | 99.8214 | 99.943 | 56.667 |
| Control group 19 | 99.9286 | 99.936 | 95.533 |
| Control group 20 | 99.8714 | 99.929 | 95.200 |

### Experimental Example 3. Confirmation of antibacterial effect of preservative for an externally-applied skin preparation in the presence of anionic surfactant

The antibacterial effect of the prepared preservatives for an externally-applied skin preparation of Examples 1 to 10 was confirmed in the presence of an anionic surfactant. For use as an anionic surfactant, sodium laureth sulfate which is the most commonly used anionic surfactant in the cosmetics field was used.

In detail, as shown in Tables 9 and 10, each of the prepared preservatives for an externally-applied skin preparation of Examples 1 to 10 was mixed with an anionic surfactant, and then, it was confirmed whether the resulting mixture has an antibacterial effect against bacteria, yeast, and molds. Specific experimental protocols are the same as those performed in Experimental Example 1.

An aqueous solution containing only an anionic surfactant at a concentration of about 30 % without adding any preservative for an externally-applied skin preparation prepared according to Examples 1 to 10 and Comparative Examples 1 to 8 was used as Control group 21. Also, an aqueous solution containing only the preservative for an externally-applied skin preparation prepared according to Comparative Example 1 at a concentration of 1 % without adding any anionic surfactant was used as Control group 22. Furthermore, each of the preservatives for an externally-applied skin preparation prepared according to Comparative Examples 1 to 8 was mixed with an anionic surfactant, and these mixtures were used as Control groups 23 to 30, respectively.

**[Table 9]**

| Ingredient (unit: weight%) | | Mixture | | | | | | | | | | | | |
|---|---|---|---|---|---|---|---|---|---|---|---|---|---|---|
| | | Control group Experimental group | | | | | | | | | | | | |
| | | 21 | 22 | 23 | 21 | 22 | 23 | 24 | 25 | 26 | 27 | 28 | 29 | 30 |
| Distilled water | | Upto 100 | | | | | | | | | | | | |
| Anionic surfactant | Sodium laureth sulfate | 30 | - | 30 | 30 | 30 | 30 | 30 | 30 | 30 | 30 | 30 | 30 | 30 |
| Comparative Example | 1 | - | 1 | 1 | - | - | - | - | - | - | - | - | - | - |
| | 1 | - | - | - | 1 | - | - | - | - | - | - | - | - | - |
| | 2 | - | - | - | - | 1 | - | - | - | - | - | - | - | - |
| | 3 | - | - | - | - | - | 1 | - | - | - | - | - | - | - |
| | 4 | - | - | - | - | - | - | 1 | - | - | - | - | - | - |
| Example | 5 | - | - | - | - | - | - | - | 1 | - | - | - | - | - |
| | 6 | - | - | - | - | - | - | - | - | 1 | - | - | - | - |
| | 7 | - | - | - | - | - | - | - | - | - | 1 | - | - | - |
| | 8 | - | - | - | - | - | - | - | - | - | - | 1 | - | - |
| | 9 | - | - | - | - | - | - | - | - | - | - | - | 1 | - |
| | 10 | - | - | - | - | - | - | - | - | - | - | - | - | 1 |

**[Table 10]**

| Ingredient (unit: weight%) | | Mixture | | | | | | |
|---|---|---|---|---|---|---|---|---|
| | | Control group | | | | | | |
| | | 24 | 25 | 26 | 27 | 28 | 29 | 30 |
| Distilled water | | Upto 100 | | | | | | |
| Anionic surfactant | Sodium laureth sulfate | 30 | 30 | 30 | 30 | 30 | 30 | 30 |
| Comparative Example | 2 | 1 | - | - | - | - | - | - |
| | 3 | - | 1 | - | - | - | - | - |
| | 4 | - | - | 1 | - | - | - | - |
| | 5 | - | - | - | 1 | - | - | - |
| | 6 | - | - | - | - | 1 | - | - |
| | 7 | - | - | - | - | - | 1 | - |
| | 8 | - | - | - | - | - | - | 1 |

Consequently, as shown in Table 11, it was confirmed that the antibacterial effect against both bacteria and fungi (yeast and molds) was at a significantly low level when only the anionic surfactant was present (Control group 21). Also, Comparative Example 1 including 4'-HAP and caprylyl glycol was found to exhibit the antibacterial effect moderately in an environment where an anionic surfactant was absent (Control group 22), whereas it exhibited the significantly reduced antibacterial effect in an environment where an anionic surfactant was present (Control group 23). Meanwhile, Examples 1 to 10 further including CHA in addition to Comparative Example 1 were found to exhibit the significantly excellent antibacterial effect with a death rate of about 99 % or more against both bacteria and fungi (yeast and molds), even in an environment where an anionic surfactant was present (Experimental groups 21 to 30). In particular, among Examples, Examples 9 and 10 further including EDTA 2Na were found to exhibit the most excellent antibacterial effect with a death rate of about 99.99 % against both bacteria and fungi (yeast and molds), even in an environment where an anionic surfactant was present (Experimental groups 29 and 30). Also, Comparative Examples 2 and 3 including CHA and any one of 4'-HAP and caprylyl glycol were found to exhibit the significantly reduced antibacterial effect in an environment where an anionic surfactant was present (Control groups 24 and 25).

Furthermore, as shown in Table 11, only when CHA, 4'-HAP, and caprylyl glycol were included at a certain content, it was confirmed that the excellent antibacterial effect was exhibited against both bacteria and fungi (yeast and molds), even in an environment where an anionic surfactant was present. In detail, only the preservative for an externally-applied skin preparation of Example including about 5 to 15 wt% of CHA, about 25 to 30 wt% of 4'-HAP, and about 13 to 15 wt% of caprylyl glycol was found to exhibit the excellent antibacterial effect against both bacteria and fungi (yeast and molds), even in an environment where an anionic surfactant was present. Meanwhile, Comparative Example in which the contents of CHA, 4'-HAP, and caprylyl glycol were beyond the aforementioned numerical ranges was found to exhibit the significantly reduced antibacterial effect in an environment where an anionic surfactant was present.

**[Table 11]**

| Death rate (%) | | | |
|---|---|---|---|
| | Bacteria | Yeast | Mold |
| Control group 21 | 56.4286 | 68.214 | 49.333 |
| Control group 22 | 99.9850 | 99.964 | 98.92 |
| Control group 23 | 50.9970 | 90.958 | 67.400 |
| Experimental group 21 | 99.9750 | 99.964 | 99.200 |
| Experimental group 22 | 99.9757 | 99.957 | 99.400 |
| Experimental group 23 | 99.9779 | 99.986 | 99.333 |
| Experimental group 24 | 99.9921 | 99.985 | 99.340 |
| Experimental group 25 | 99.9950 | 99.992 | 99.767 |
| Experimental group 26 | 99.9943 | 99.993 | 99.773 |
| Experimental group 27 | 99.9994 | 99.994 | 99.997 |
| Experimental group 28 | 99.9996 | 99.995 | 99.997 |
| Experimental group 29 | 99.9999 | 99.999 | 99.999 |
| Experimental group 30 | 99.9999 | 99.999 | 99.999 |
| Control group 24 | 56.4286 | 98.214 | 69.333 |
| Control group 25 | 60.7143 | 99.286 | 73.333 |
| Control group 26 | 56.4286 | 98,500 | 88.000 |
| Control group 27 | 60.7143 | 97.143 | 88.667 |
| Control group 28 | 64.2857 | 98.714 | 82.667 |
| Control group 29 | 87.1429 | 97.929 | 70.667 |
| Control group 30 | 90.7143 | 92.857 | 77.333 |

### Experimental Example 4. Confirmation of antibacterial effect of preservative for an externally-applied skin preparation in the presence of zwitterionic surfactant

The antibacterial effect of the prepared preservatives for an externally-applied skin preparation of Examples 1 to 10 was confirmed in the presence of a zwitterionic surfactant. For use as a zwitterionic surfactant, cocamidopropyl betaine which is the most commonly used zwitterionic surfactant in the cosmetic field was used.

In detail, as shown in Tables 12 and 13, each of the prepared preservatives for an externally-applied skin preparation of Examples 1 to 10 was mixed with a zwitterionic surfactant, and then, it was confirmed whether the resulting mixture has an antibacterial effect against bacteria, yeast, and molds. Specific experimental protocols are the same as those performed in Experimental Example 1.

An aqueous solution containing only a zwitterionic surfactant at a concentration of about 30 % without adding any preservative for an externally-applied skin preparation prepared according to Examples 1 to 10 and Comparative Examples 1 to 8 was used as Control group 31. Also, an aqueous solution containing only the preservative for an externally-applied skin preparation prepared according to Comparative Example 1 at a concentration of 1 % without adding any zwitterionic surfactant was used as Control group 32. Furthermore, each of the preservatives for an externally-applied skin preparation prepared according to Comparative Examples 1 to 8 was mixed with a zwitterionic surfactant, and these mixtures were used as Control groups 33 to 40, respectively.

**[Table 12]**

| Ingredient (unit: weight%) | | Mixture | | | | | | | | | | | | |
|---|---|---|---|---|---|---|---|---|---|---|---|---|---|---|
| | | Control group Experimental group | | | | | | | | | | | | |
| | | 31 | 32 | 33 | 31 | 32 | 33 | 34 | 35 | 36 | 37 | 38 | 39 | 40 |
| Distilled water | | UF10 100 | | | | | | | | | | | | |
| Zwitterionic surfactant | Cocamidopropyl betaine | 30 | - | 30 | 30 | 30 | 30 | 30 | 30 | 30 | 30 | 30 | 30 | 30 |
| Comparative Example | 1 | - | 1 | 1 | - | - | - | - | - | - | - | - | - | - |
| Example | 1 | - | - | - | 1 | - | - | - | - | - | - | - | - | - |
| | 2 | - | - | - | - | 1 | - | - | - | - | - | - | - | - |
| | 3 | - | - | - | - | - | 1 | - | - | - | - | - | - | - |
| | 4 | - | - | - | - | - | - | 1 | - | - | - | - | - | - |
| | 5 | - | - | - | - | - | - | - | 1 | - | - | - | - | - |
| | 6 | - | - | - | - | - | - | - | - | 1 | - | - | - | - |
| | 7 | - | - | - | - | - | - | - | - | - | 1 | - | - | - |
| | 8 | - | - | - | - | - | - | - | - | - | - | 1 | - | - |
| | 9 | - | - | - | - | - | - | - | - | - | - | - | 1 | - |
| | 10 | - | - | - | - | - | - | - | - | - | - | - | - | 1 |

**[Table 13]**

| Ingredient (unit: weight%) | | Mixture | | | | | | |
|---|---|---|---|---|---|---|---|---|
| | | Control group | | | | | | |
| | | 34 | 35 | 36 | 37 | 38 | 39 | 40 |
| Distilled water | | Upto 100 | | | | | | |
| Zwitterionic surfactant | Cocamidopropyl betaine | 30 | 30 | 30 | 30 | 30 | 30 | 30 |
| Comparative Example | 2 | 1 | - | - | - | - | - | - |
| | 3 | - | 1 | - | - | - | - | - |
| | 4 | - | - | 1 | - | - | - | - |
| | 5 | - | - | - | 1 | - | - | - |
| | 6 | - | - | - | - | 1 | - | - |
| | *1* | - | - | - | - | - | 1 | - |
| | 8 | - | - | - | - | - | - | 1 |

Consequently, as shown in Table 14, it was confirmed that the antibacterial effect against both bacteria and fungi (yeast and molds) was at a significantly low level when only the zwitterionic surfactant was present (Control group 31). Also, Comparative Example 1 including 4'-HAP and caprylyl glycol was found to exhibit the antibacterial effect moderately in an environment where a surfactant was absent (Control group 32), whereas it exhibited significantly reduced antibacterial effect in an environment where a zwitterionic surfactant was present (Control group 33). Meanwhile, Examples 1 to 10 further including CHA in addition to Comparative Example 1 were found to exhibit the significantly excellent antibacterial effect with a death rate of about 99 % or more against both bacteria and fungi (yeast and molds), even in an environment where a zwitterionic surfactant was present (Experimental groups 31 to 40). In particular, among Examples, Examples 9 and 10 further including EDTA 2Na were found to exhibit the most excellent antibacterial effect with a death rate of about 99.99 % against both bacteria and fungi (yeast and molds), even in an environment where a zwitterionic surfactant was present (Experimental groups 33 and 40). Also, Comparative Examples 2 and 3 including CHA and any one of 4'-HAP and caprylyl glycol were found to exhibit the significantly reduced antibacterial effect (especially against fungi (yeast and molds)), in an environment where a zwitterionic surfactant was present (Control groups 34 and 35).

Furthermore, as shown in Table 14, only when CHA, 4'-HAP, and caprylyl glycol were included at a certain content, it was confirmed that the excellent antibacterial effect was exhibited against both bacteria and fungi (yeast and molds), even in an environment where a zwitterionic surfactant was present. In detail, only the preservative for an externally-applied skin preparation of Example including about 5 to 15 wt% of CHA, about 25 to 30 wt% of 4'-HAP, and about 13 to 15 wt% of caprylyl glycol was found to exhibit the excellent antibacterial effect against both bacteria and fungi (yeast and molds), even in an environment where a zwitterionic surfactant was present. Meanwhile, Comparative Example in which the contents of CHA, 4'-HAP, and caprylyl glycol were beyond the aforementioned numerical ranges was found to exhibit the significantly reduced antibacterial effect (especially, against fungi (yeast and molds)), in an environment where a zwitterionic surfactant was present.

**[Table 14]**

| Death rate (%) | | | |
|---|---|---|---|
| | Bacteria | Yeast | Mold |
| Control group 31 | 69.9943 | 88.214 | 59.993 |
| Control group 32 | 99.9850 | 99.964 | 98.92 |
| Control group 33 | 79.9970 | 95.958 | 79.991 |
| Experimental group 31 | 99.9750 | 99.971 | 99.833 |
| Experimental group 32 | 99.9757 | 99.971 | 99.847 |
| Experimental group 33 | 99.9779 | 99.993 | 99.933 |
| Experimental group 34 | 99.9921 | 99.993 | 99.934 |
| Experimental group 35 | 99.9950 | 99.997 | 99.953 |
| Experimental group 36 | 99.9943 | 99.997 | 99.960 |
| Experimental group 37 | 99.9994 | 99.999 | 99.993 |
| Experimental group 38 | 99.9996 | 99.999 | 99.993 |
| Experimental group 39 | 99.9999 | 99.999 | 99.999 |
| Experimental group 40 | 99.9999 | 99.999 | 99.999 |
| Control group 34 | 99.9564 | 97.929 | 89.333 |
| Control group 35 | 99.9579 | 99.214 | 90.667 |
| Control group 36 | 99.9564 | 99.143 | 94.400 |
| Control group 37 | 99.9636 | 99.071 | 95.267 |
| Control group 38 | 99.9643 | 98.214 | 95.867 |
| Control group 39 | 99.9871 | 97.929 | 96.800 |
| Control group 40 | 99.9779 | 92.857 | 97.133 |

### 2. Preparation and evaluation of cosmetic composition including preservative for externally-applied skin preparation

### Example 11. - Example 20. Preparation of cosmetic composition including preservative for externally-applied skin preparation

In this example, a cosmetic composition formulated into a shampoo was prepared, including the preservatives for an externally-applied skin preparation prepared according to Examples 1 to 10 and additionally including a surfactant and other cosmetic raw materials.

In detail, each of the preservatives for an externally-applied skin preparation prepared according to Examples 1 to 10 was mixed and stirred with a surfactant and other cosmetic raw materials., so as to obtain a shampoo-type cosmetic composition. Here, the ingredients and weight% of the preservative for an externally-applied skin preparation, surfactant, and other cosmetic raw materials were as shown in Table 15. Also, as shown in Table 16, a shampoo-type cosmetic composition including any one of the preservatives for an externally-applied skin preparation prepared according to Comparative Examples 1 to 8 and additionally including a surfactant and other cosmetic raw materials was prepared, and used as Comparative Example.

**[Table 15]**

| Ingredient (unit: weight%) | | Formulation Examples | | | | | | | | | |
|---|---|---|---|---|---|---|---|---|---|---|---|
| | | Example 11 | Example 12 | Example 13 | Example 14 | Example 15 | Example 16 | Example 17 | Example 18 | Example 13 | Example 20 |
| Distilled water | | Upto 100 | | | | | | | | | |
| Glycerin | | 5 | 5 | 5 | 5 | 5 | 5 | 5 | 5 | 5 | 5 |
| Polyquaternium-67 | | 2 | 2 | 2 | 2 | 2 | 2 | 2 | 2 | 2 | 2 |
| Coco-Betaine | | 8 | 8 | 8 | 8 | 8 | 8 | 8 | 8 | 8 | 8 |
| Sodium laureth sulfate | | 10 | 10 | 10 | 10 | 10 | 10 | 10 | 10 | 10 | 10 |
| Lauryl glucoside | | 7 | 7 | 7 | 7 | 7 | 7 | 7 | 7 | 7 | 7 |
| Cocamidopropyl betaine | | 6 | 6 | 6 | 6 | 6 | 6 | 6 | 6 | 6 | 6 |
| Sodium chloride | | 0.5 | 0.5 | 0.5 | 0.5 | 0.5 | 0.5 | 0.5 | 0.5 | 0.5 | 0.5 |
| | Example 1 | 1 | - | - | - | - | - | - | - | - | - |
| | Example 2 | - | 1 | - | - | - | - | - | - | - | - |
| | Example 3 | - | - | 1 | | - | - | - | - | - | - |
| | Example 4 | - | - | - | 1 | - | - | - | - | - | - |
| Preservative for externally-applied skin preparation | Example 5 | - | - | - | - | 1 | - | - | - | - | - |
| | Example 6 | - | - | - | - | - | 1 | - | - | - | - |
| | Example 7 | - | - | - | - | - | - | 1 | - | - | - |
| | Example 8 | - | - | - | - | - | - | - | 1 | - | - |
| | Example 9 | - | - | - | - | - | - | - | - | 1 | |
| | Example 10 | - | - | - | - | - | - | - | - | - | 1 |

**[Table 16]**

| Ingredient (unit: weight%) | Formulation Example | | | | | | | | |
|---|---|---|---|---|---|---|---|---|---|
| | Comparative Example 9 | Comparative Example 10 | Comparative Example 11 | Comparative Example 12 | Comparative Example 13 | Comparative Example 14 | Comparative Example 15 | Comparative Example 16 | |
| Distilled water | Upto 100 | | | | | | | | |
| Glycerin | 5 | 5 | 5 | 5 | 5 | 5 | 5 | 5 | |
| Polyquaternium-67 | 2 | 2 | 2 | 2 | 2 | 2 | 2 | 2 | |
| Coco-Betaine | 8 | 8 | 8 | 8 | 8 | 8 | a | 8 | |
| Sodium laureth sulfate | 10 | 10 | 10 | 1D | 10 | 10 | 10 | 10 | |
| Lauryl glucoside | 7 | 7 | 7 | 7 | 7 | 7 | 7 | 7 | |
| Cocamidopropyl betaine | | 6 | 6 | 6 | 6 | 6 | 6 | 6 | 6 |
| Sodium chloride | | 0.5 | 0,5 | 0.5 | 0.5 | 0.5 | 0.5 | 0.5 | 0.5 |
| Preservative for externally-applied skin preparation | Comparative Example 1 | 1 | - | - | - | - | - | - | - |
| | Comparative Example 2 | - | 1 | - | - | - | - | - | - |
| | Comparative Example 3 | - | - | 1 | - | - | - | - | - |
| | Comparative Example 4 | - | - | - | 1 | - | - | - | - |
| | Comparative Example 5 | - | - | - | - | 1 | - | - | - |
| | Comparative Example 6 | - | - | - | - | - | 1 | - | - |
| | Comparative Example 7 | - | - | - | - | - | - | 1 | - |
| | Comparative Example 8 | - | - | - | - | - | - | - | 1 |

### Experimental Example 5. Confirmation of antiseptic activity of cosmetic composition including preservative for externally-applied skin preparation

To evaluate the antiseptic activity of the preservatives for an externally-applied skin preparation prepared according to Examples 1 to 10, a certain number of bacteria was inoculated into the cosmetic compositions including the preservative for an externally-applied skin preparation, i.e., the shampoo-type compositions prepared according to Examples 11 to 20 to confirm the number of viable bacteria. Here, the cosmetic compositions prepared according to Comparative Examples 9 to 16 were used as Comparative Examples.

In detail, each of the cosmetic compositions prepared according to Examples 11 to 20 and Comparative Examples 9 to 16 was inoculated with a mixed bacterial culture, i.e., cultures of *E*. *coli* (ATCC 8739), *P. aeruginosa* (ATCC 9027), and *S. aureus* (ATCC 6538) mixed at a ratio of 1:1:1. Here, the inoculation was done so that the initial concentration of bacteria per sample was about 10⁶ CFU/g. Afterwards, each sample was cultured in a thermostatic bath at about 25 °C for about 4 weeks. In the meantime, about 1 g of each sample was taken at about 7-day intervals to measure the number of viable bacteria.

Also, each of the cosmetic compositions prepared according to Examples 11 to 20 and Comparative Examples 9 to 16 was inoculated with a yeast culture, i.e., a culture of *Candida albicans* (ATCC 10231). Here, the inoculation was done so that the initial concentration of bacteria per sample was about 10⁵ CFU/g. Afterwards, each sample was cultured in a thermostatic bath at about 25 °C for about 4 weeks. In the meantime, about 1 g of each sample was taken at about 7-day intervals to measure the number of viable bacteria.

Furthermore, each of the cosmetic compositions prepared according to Examples 11 to 20 and Comparative Examples 9 to 16 was inoculated with a mold culture, i.e., a culture of *Aspergillus brasiliensis* (ATCC 16404). Here, the inoculation was done so that the initial concentration of bacteria per sample was about 10⁵CFU/g. Afterwards, each sample was cultured in a thermostatic bath at about 25 °C for about 4 weeks. In the meantime, about 1 g of each sample was taken at about 7-day intervals to measure the number of viable bacteria.

Consequently, as shown in Table 17, the preservatives for an externally-applied skin preparation of Examples including CHA, 4'-HAP, and caprylyl glycol were found to exhibit an excellent antiseptic affect against both bacteria and fungi (yeast and molds) when the formulation of the preservatives was a cosmetic composition including various surfactants. Meanwhile, the preservatives for an externally-applied skin preparation of Comparative Examples 1 to 3 including two selected from CHA, 4'-HAP, and caprylyl glycol were found to exhibit a significantly reduced antiseptic effect against bacteria and fungi (yeast and molds) (especially against molds) when the formulation of the preservatives was a cosmetic composition including various surfactants.

Furthermore, as shown in Table 17, only when CHA, 4'-HAP, and caprylyl glycol were included at a certain content, it was confirmed that an excellent antiseptic effect was exhibited against both bacteria and fungi (yeast and molds) when the formulation of the preservatives was a cosmetic composition including various surfactants. In detail, only the preservative for an externally-applied skin preparation of Example including about 5 to 15 wt% of CHA, about 25 to 30 wt% of 4'-HAP, and about 13 to 15 wt% of caprylyl glycol was found to exhibit an excellent antiseptic effect against both bacteria and fungi (yeast and molds) when the formulation of the preservatives was a cosmetic composition including various surfactants. Meanwhile, the preservatives for an externally-applied skin preparation of Comparative Examples 4 to 8 including CHA, 4'-HAP, and caprylyl glycol in the contents beyond the aforementioned numerical ranges were found to exhibit a significantly reduced antiseptic effect against bacteria and fungi (yeast and molds) (especially against molds) when the formulation of the preservatives was a cosmetic composition including various surfactants.

**[Table 17]**

| | | Day 7 | | | Day 14 | | | Day 28 | | |
|---|---|---|---|---|---|---|---|---|---|---|
| Formulation Example | Preservative for externally-applied skin preparation | Bacteria | Yeast | Mold | Bacteria | Yeast | Mold | Bacteria | Yeast | Mold |
| Example 11 | Example 1 | 30 | <10 | 200 | <10 | <10 | 50 | <10 | <10 | <10 |
| Example 12 | Example 2 | 40 | <10 | 210 | <10 | <10 | 40 | <10 | <10 | <10 |
| Example 13 | Example 3 | 20 | <10 | 130 | <10 | <10 | 12 | <10 | <10 | <10 |
| Example 14 | Example 4 | 20 | <10 | 50 | <10 | <10 | 10 | <10 | <10 | <10 |
| Example 15 | Example 5 | <10 | <10 | 50 | <10 | <10 | <10 | <10 | <10 | <10 |
| Example 16 | Example 6 | <10 | <10 | 50 | <10 | <10 | <10 | <10 | <10 | <10 |
| Example 17 | Example 7 | <10 | <10 | 32 | <10 | <10 | <10 | <10 | <10 | <10 |
| Example 18 | Example 8 | <10 | <10 | 35 | <10 | <10 | <10 | <10 | <10 | <10 |
| Example 19 | Example 9 | <10 | <10 | 20 | <10 | <10 | <10 | <10 | <10 | <10 |
| Example 20 | Example 10 | <10 | <10 | 10 | <10 | <10 | <10 | <10 | <10 | <10 |
| Comparative Example 9 | Comparative Example 1 | 900 | 400 | 10000 | 400 | <10 | 2000 | <10 | <10 | 1000 |
| Comparative Example 10 | Comparative Example 2 | 1000 | 550 | 15000 | 500 | <10 | 3100 | <10 | <10 | 1800 |
| Comparative Example 11 | Comparative Example 3 | 1100 | 490 | 11000 | 400 | <10 | 3300 | <10 | <10 | 1400 |
| Comparative Example 12 | Comparative Example 4 | 80 | 140 | 5500 | 50 | <10 | 1100 | <10 | <10 | 800 |
| Comparative Example 13 | Comparative Example 5 | 100 | 150 | 4600 | 50 | <10 | 1000 | <10 | <10 | 700 |
| Comparative Example 14 | Comparative Example 6 | 300 | 190 | 2100 | 100 | <10 | 600 | <10 | <10 | 200 |
| Comparative Example 15 | Comparative Example 7 | 120 | 300 | 1000 | 60 | <10 | 90 | <10 | <10 | 50 |
| Comparative Example 16 | Comparative Example 8 | 150 | 340 | 1500 | 50 | <10 | 120 | <10 | <10 | 50 |

Through Examples and Experimental Examples, it was confirmed that even the preservatives for an externally-applied skin preparation including 4'-HAP, caprylyl glycol, and dipropylene glycol and exhibiting the antibacterial effect showed significant reduction in the antibacterial and antiseptic effects in an environment where a surfactant was present. However, it was also confirmed that, when the preservatives for an externally-applied skin preparation of Examples additionally included CHA, such problems reduced effectiveness were solved and excellent antibacterial and antiseptic effects against both bacteria and fungi (yeast and molds) were exhibited in the cosmetic compositions including various surfactants. Also, it was confirmed that the preservatives for an externally-applied skin preparation of Examples additionally including EDTA 2Na exhibited the most excellent antibacterial and antiseptic effects against both bacteria and fungi (yeast and molds) in the cosmetic compositions including various surfactants. In particular, the inclusion of CHA in the preservative for an externally-applied skin preparation does not necessarily mean that the excellent antibacterial effect is exhibited in the cosmetic composition including various surfactants, and only the preservatives for an externally-applied skin preparation of Examples including all of the ingredients of CHA, 4'-HAP, and caprylyl glycol were found to exhibit excellent antibacterial and antiseptic effects against both bacteria and fungi (yeast and molds) in the cosmetic compositions including various surfactants.

Furthermore, through Examples and Experimental Examples, only the preservatives for an externally-applied skin preparation including about 5 to 15 wt% of CHA , about 25 to 30 wt% of 4'-HAP, and about 13 to 15 wt% of caprylyl glycol were found to exhibit excellent antibacterial and antiseptic effects against both bacteria and fungi (yeast and molds) in the cosmetic compositions including various surfactants, but those including CHA, 4'-HAP, and caprylyl glycol in the contents beyond the aforementioned numerical values were found to exhibit significantly reduced antibacterial and antiseptic effects against bacteria and fungi (yeast and molds) (especially against molds) in the cosmetic compositions including various surfactants.

Therefore, the preservative for an externally-applied skin preparation including specific ingredients and contents according to an aspect was found to be utilized as a preservative, an antiseptic agent, or a preservative supplement that can exhibit excellent antibacterial and antiseptic effects in cosmetic compositions including various surfactants (e.g., a cleansing cosmetic composition, etc.).

While the foregoing has described certain aspects of the present disclosure, it will be apparent to those skilled in the art that these specific techniques are merely preferred embodiments and are not intended to limit the scope of the present disclosure. Therefore, the substantial scope of the present disclosure will be defined by the appended claims and equivalents thereof.

## Claims

1. A preservative for an externally-applied skin preparation, comprising: caprylic hydroxamic acid (CHA); 4'-hydroxyacetophenone (4'-HAP); and caprylyl glycol.

2. The preservative of claim 1, further comprising one or more polyols selected from the group consisting of glycerin, ethylene glycol, diethylene glycol, propylene glycol, dipropylene glycol, butylene glycol, pentylene glycol, hexylene glycol, ethoxydiglycol, and C3-C12 alkanediol.

3. The preservative of claim 1, wherein the preservative for an externally-applied skin preparation comprises 5 to 15 wt% of the CHA, based on a total weight of the preservative for an externally-applied skin preparation.

4. The preservative of claim 1, wherein the preservative for an externally-applied skin preparation comprises 25 to 30 wt% of the 4'-HAP, based on a total weight of the preservative for an externally-applied skin preparation.

5. The preservative of claim 1, wherein the preservative for an externally-applied skin preparation comprises 13 to 15 wt% of the caprylyl glycol, based on a total weight of the preservative for an externally-applied skin preparation.

6. The preservative of claim 1, wherein a weight ratio of the CHA : the 4'-HAP : the caprylyl glycol is 5 to 15 : 25 to 30 : 13 to 15.

7. The preservative of claim 1, further comprising EDTA 2Na.

8. The preservative of claim 1, the externally-applied skin preparation comprises a surfactant.

9. The preservative of claim 8, wherein the externally-applied skin preparation is a cleansing cosmetic composition.

10. A cosmetic composition comprising the preservative of any one of claims 1 to 9.

11. The cosmetic composition of claim 10, wherein the cosmetic composition further comprises a surfactant.

12. The cosmetic composition of claim 11, wherein the cosmetic composition is a cleansing cosmetic composition.

13. The cosmetic composition of claim 10, wherein the cosmetic composition comprises 0.0001 wt% to 50 wt% of the preservative, based on a total weight of the cosmetic composition.
